# EUROPEAN PATENT SPECIFICATION

(11) **EP 3 039 122 B1**
(45) Date of publication and mention of the grant of the patent: **17.07.2019**
(21) Application number: 14761822.7
(22) Date of filing: 29.08.2014
(51) Int. Cl.: C12N 5/071

(54) **GENERATION OF ENDOCRINE PROGENITOR CELLS FROM HUMAN PLURIPOTENT STEM CELLS USING SMALL MOLECULES**
ERZEUGUNG VON ENDOKRINEN VORLÄUFERZELLEN AUS MENSCHLICHEN PLURIPOTENTEN STAMMZELLEN MIT KLEINEN MOLEKÜLEN
GÉNÉRATION DE CELLULES PROGÉNITRICES ENDOCRINES À PARTIR DE CELLULES SOUCHES PLURIPOTENTES HUMAINES À L'AIDE DE PETITES MOLÉCULES

(30) Priority: 30.08.2013 EP 13182440; 09.09.2013 US 201361875191 P
(43) Date of publication of application: 06.07.2016
(73) Proprietor: Novo Nordisk A/S, 2880 Bagsværd (DK); Takara Bio Europe AB, 413 46 Göteborg (SE)
(72) Inventor: DØHN, Ulrik, DK-2880 Bagsværd (DK); CHRISTOPHERSEN, Nicolaj Strøyer, DK-2880 Bagsværd (DK); EKBERG, Jenny, DK-2880 Bagsværd (DK)
(86) International application number: PCT/EP2014/068393
(87) International publication number: WO 2015/028614

(56) References cited:
- EP-A1- 2 505 639
- WO-A2-2007/103282
- US-A1- 2009 004 152
- YUYA KUNISADA ET AL: "Small molecules induce efficient differentiation into insulin-producing cells from human induced pluripotent stem cells", STEM CELL RESEARCH, vol. 8, no. 2, 1 March 2012 (2012-03-01), pages 274-284, XP055094959, ISSN: 1873-5061, DOI: 10.1016/j.scr.2011.10.002 cited in the application
- JIANG J ET AL: "Generation of insulin-producing islet-like clusters from human embryonic stem cells", STEM CELLS, ALPHAMED PRESS, DAYTON, OH, US, vol. 25, no. 8, 1 August 2007 (2007-08-01) , pages 1940-1953, XP008084029, ISSN: 1066-5099
- M. C. NOSTRO ET AL: "Stage-specific signaling through TGF family members and WNT regulates patterning and pancreatic specification of human pluripotent stem cells", DEVELOPMENT, vol. 138, no. 5, 1 March 2011 (2011-03-01) , pages 861-871, XP055063429, ISSN: 0950-1991, DOI: 10.1242/dev.055236 cited in the application

## Description

### TECHNICAL FIELD

The present invention relates to methods of generating endocrine progenitor cells from human pluripotent stem cells, such as human embryonic stem cells and induced pluripotent stem cells.

### BACKGROUND OF THE INVENTION

Beta-cell transplantation potentially provides the ultimate cure for type I diabetes. However, the limited availability of donor beta-cells constrains the use of this treatment as a clinical therapy.

Pluripotent stem (PS) cells can proliferate infinitely and differentiate into many cell types; thus, PS cells are a promising source for beta-cells. However, before PS cells can be used to treat diabetes, they need to be efficiently and reproducibly differentiated to pancreatic beta-cells. During vertebrate embryonic development, a pluripotent cell gives rise to the three germ layers; ectoderm, mesoderm and endoderm.

Induction of definitive endoderm (DE) is the first step towards formation of endoderm derived tissues, such as pancreatic tissue. Generation of pancreatic endoderm (PE) from DE cells is necessary for the generation of insulin-producing beta-cells. PE cells with the potential to become endocrine progenitors (EP) are characterized by co-expression of two important transcription factors, PDX1 and NKX6.1.

Stepwise *in vitro* differentiation protocols have been established for generating pancreatic cells from PS cells.

These protocols generally mimic the major events of pancreatic development, which includes several stages such as formation of the DE, primitive gut, posterior foregut, PE, EP and ultimately the fully differentiated pancreatic beta-cells.

A protocol for obtaining pancreatic(-like) cells from human embryonic stem (hES) cells and induced pluripotent stem (iPS) cells is exemplified by the protocols described in several scientific articles (Aoi *et al.* 2008; D'Amour *et al.* 2006; Jiang *et al.* 2007; Kroon *et al.* 2008; Takahashi *et al.* 2007; Takahashi & Yamanaka 2006; and Wernig *et al.* 2007)).

To date, efficient DE differentiation of hES cells has been achieved by activin A and Wnt treatment. DE cells can be further differentiated into PE cells using retinoic acid (RA) (Cai *et al.* 2010; D'Amour *et al.* 2006) and BMP inhibition (Kunisada et *al*. 2012; Schulz *et al.* 2012; Zhang *et al.*(2009).

Following the generation of PE cells the next step in the route of generating pancreatic beta-cells is to generate EP cells that express the NGN3 and NKX2.2 markers.

Nostro *et al.* (2012) and Kunisada *et al.* (2011) describe methods for differentiating PE to EP.

However, there is a need for a more efficient method of differentiating PE to EP.

### SUMMARY OF THE INVENTION

The present invention provide improved methods for differentiating pancreatic endoderm (PE) into endocrine progenitor (EP) cells by combining features from known protocols thereby increasing the percentage of NGN3/NKX2.2 double positive cells. By addition of small molecules to the aforementioned method said percentage can be further increased. Certain combinations of small molecules allow for a further advantageous increase in the percentage of NGN3/NKX2.2 double positives.

The present description further relates to EP cells obtainable by the methods of the present invention.

The present description further relates to medical use of said cells *inter alia* in the treatment of type I diabetes.

The present invention takes an alternative approach to improve the efficiency of differentiating human PE cells toward fully differentiated beta-cells, by providing a method to increase the percentage of NGN3/NKX2.2 double positive cells, a hallmark for EP cells committed to an endocrine cell fate.

In one embodiment the invention provides an improved pancreatic beta-cell precursor population, i.e. EP cells with increased percentage of NGN3/NKX2.2 double positive cells.

In another embodiment the present invention provides a more homogenous EP cell population, which is important for the further development of these cells towards fully differentiated pancreatic beta-cells.

In another embodiment the present invention also provides a more synchronised EP population to get to the next stage of differentiation, namely the glucose responsive fully differentiated beta-cells.

In one aspect the present invention provides a method for obtaining NGN3/NKX2.2 double positive endocrine progenitor cells wherein a cell population comprising pancreatic endoderm cells are exposed to a TGF-β type I receptor inhibitor and a BMP antagonist and an adenylate cyclase activator and nicotinamide in basal medium.

In one aspect the present invention provides a method for obtaining NGN3/NKX2.2 double positive endocrine progenitor cells wherein a cell population comprising pancreatic endoderm cells are exposed to gefitinib, JNK inhibitor VIII and DAPT.

The invention may also solve further problems that will be apparent from the disclosure of the exemplary embodiments.

### BRIEF DESCRIPTION OF DRAWINGS

**Fig. 1** shows the advantageous effect of a method of the present invention in NGN3 mRNA induction.
**Fig. 2** shows the advantageous effect of a method of the present invention in generating NGN3/NKX2.2 double positive endocrine progenitor cells.
**Fig. 3** shows the individual effects and advantageous effects of combining small molecules of the present invention.
**Fig. 4** shows the individual effects and advantageous effects of combining several small molecules of the present invention.

### DETAILED DESCRIPTION

The present invention related to methods of generating endocrine progenitor (EP) cells from pluripotent stem cells, such as embryonic stem (ES) cells and induced pluripotent stem cells of a human origin.

Stem cells are undifferentiated cells defined by their ability at the single cell level to both self-renew and differentiate to produce progeny cells, including self-renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation.

Stem cells are classified by their developmental potential as: (1) totipotent, meaning able to give rise to all embryonic and extraembryonic cell types; (2) pluripotent, meaning able to give rise to all embryonic cell types; (3) multi-potent, meaning able to give rise to a subset of cell lineages, but all within a particular tissue, organ, or physiological system (for example, hematopoietic stem cells (HSC) can produce progeny that include HSC (self-renewal), blood cell restricted oligopotent progenitors and all cell types and elements (e.g., platelets) that are normal components of the blood); (4) oligopotent, meaning able to give rise to a more restricted subset of cell lineages than multi-potent stem cells; and (5) unipotent, meaning able to give rise to a single cell lineage (e.g., spermatogenic stem cells).

Mature or differentiated pancreatic cells do not proliferate and do secrete high levels of pancreatic endocrine hormones or digestive enzymes. E.g., fully differentiated beta-cells secrete insulin at high levels in response to glucose. Changes in cell interaction and maturation occur as cells lose markers of undifferentiated cells or gain markers of differentiated cells. Loss or gain of a single marker can indicate that a cell has "matured or fully differentiated".

The present invention takes an alternative approach to improve the efficiency of differentiating human PE cells toward fully differentiated beta-cells, by providing a method to improve the percentage of NGN3/NKX2.2 double positive cells, which are markers for an EP cell population, one of the cell stages necessary to arrive at an insulin producing pancreatic beta-cell.

Furthermore, the present invention provides a more homogenous and synchronised EP cell population which is important for the further development of these cells towards the insulin producing beta-cell.

The present invention may also solve further problems that will be apparent from the disclosure of the exemplary embodiments.

As used herein, "insulin producing cells" refers to cells that produce and store or secrete detectable amounts of insulin in response to glucose. "Insulin producing cells" can be individual cells or collections of cells.

As used herein the term "beta-cells" refers to cells that reside within small cell clusters called islets of Langerhans in the pancreas. Beta-cells respond to high blood glucose levels by secreting the peptide hormone insulin, which acts on other tissues to promote glucose uptake from the blood, for example in the liver where it promotes energy storage by glycogen synthesis.

As used herein "differentiate" or "differentiation" refers to a process where cells progress from an undifferentiated state to a differentiated state, from an immature state to a less immature state or from an immature state to a mature state. For example, early undifferentiated embryonic pancreatic cells are able to proliferate and express characteristics markers, like PDX1, NKX6.1 and PTF1a.

The term "differentiation factor" refers to a compound added to ES- or pancreatic precursor cells to enhance their differentiation to EP cells. Differentiation factors may also drive further differentiation into mature beta-cells.

Exemplary differentiation factors include hepatocyte growth factor, keratinocyte growth factor, exendin-4, basic fibroblast growth factor, insulin-like growth factor-1, nerve growth factor, epidermal growth factor platelet-derived growth factor, glucagon-like peptide 1, indolactam V, IDE1&2 and retinoic acid.

In some aspects differentiation of the cells comprises culturing the cells in a medium comprising one or more differentiation factors.

In one embodiment the invention relates to a method of providing pancreatic endocrine function to a mammal deficient in its production of at least one pancreatic hormone, the method comprising the steps of implanting cells obtained by any of the methods of the invention in an amount sufficient to produce a measurable amount of said at least one pancreatic hormone in said mammal.

In one embodiment an EP cell population prepared according to the methods of the present invention may be used in the treatment of diabetes, e.g. by implantation into a patient in need of such treatment.

As used herein, the term "human pluripotent stem (hPS) cells" refers to cells that may be derived from any source and that are capable, under appropriate conditions, of producing human progeny of different cell types that are derivatives of all of the 3 germinal layers (endoderm, mesoderm, and ectoderm). hPS cells have the ability to form a teratoma in 8-12 week old SCID mice and/or the ability to form identifiable cells of all three germ layers in tissue culture.

The various methods and other embodiments described herein may require or utilise hPS cells from a variety of sources.

Additionally or alternatively, suitable hPS cells may be obtained from established cell lines and/or human induced pluripotent stem (hiPS) cells.

As used herein, the term "hiPS cells" refers to human induced pluripotent stem cells. It is further envisaged that any human pluripotent stem cell in turn can be used in the present invention, including differentiated adult cells which are reprogrammed to pluripotent cells by e.g. the treating adult cells with certain transcription factors, such as OCT4, SOX2, NANOG, and LIN28.

As used herein, an "EP cell population" is a population of pancreatic beta-cell precursors in which at least 5% of the cell population are NGN3/NKX2.2 double positives.

As used herein, the term "PDX1" refers to a homeodomain transcription factor implicated in pancreas development. "NGN3" as used herein, is a member of the neurogenin family of basic loop- helix-loop transcription factors. "NKX2.2" and "NKX6.1" as used herein are members of the NKX transcription factor family. "Islet-1" or "Isl-1" as used herein is a member of the LIM/homeodomain family of transcription factors, and is expressed in the developing pancreas. "MafA" as used herein is a transcription factor expressed in the pancreas, and controls the expression of genes involved in insulin biosynthesis and secretion.

In one embodiment the present invention provides an alternative and more efficient method compared to that known in art for differentiating PE cells to EP cells thereby yielding a more homogenous EP population.

A homogenous EP population is a desirable starting point for further differentiation into fully differentiated beta-cells.

In one embodiment PE cells are treated in such a fashion that the percentage of NGN3/NKX2.2 double positives in the resulting population is higher than that achievable using known protocols for differentiating a PE cell population to EP cell population.

In one embodiment using the method of the present description a 600 fold up-regulation of NGN3 mRNA is observed when compared to treatment with basal media.

In one embodiment known methods for differentiating PE to EP cells are used to improve the percentage of NGN3/NKX2.2 double positives in the resulting EP cell population.

In one embodiment known methods for differentiating PE to EP cells are used synergistically to improve the percentage of NGN3/NKX2.2 double positives in the resulting EP cell population.

In one embodiment elements from known methods for differentiating PE to EP cells are used synergistically to improve the percentage of NGN3/NKX2.2 double positives in the resulting EP cell population.

In one embodiment, the method according to reference example 2 produces a population of endocrine progenitor cells that are at least 8% effect NGN3/NKX2.2 double positive.

In one embodiment, the method according to reference example 2 produces a population of endocrine progenitor cells that are at least 9% effect NGN3/NKX2.2 double positive.

In one embodiment, the method according to reference example 2 produces a population of endocrine progenitor cells that are at least10% effect NGN3/NKX2.2 double positive.

In one embodiment, the method according to reference example 2 produces a population of endocrine progenitor cells that are at least15% effect NGN3/NKX2.2 double positive.

In one embodiment, the method according to the present invention produces a population of endocrine progenitor cells that are 15-100% effect NGN3/NKX2.2 double positive.

In one embodiment small molecules are used to increase the percentage of NGN3/NKX2.2 double positives in a PE to EP differentiation process.

In one embodiment small molecules are used in combination to increase the percentage of NGN3/NKX2.2 double positives in a PE to EP differentiation process.

In one embodiment small molecules are used in combination to synergistically increase the percentage of NGN3/NKX2.2 double positives in a PE to EP differentiation process.

In the below embodiments a number of small molecules and concentrations are listed which are useful in promoting differentiation of PE to EP.

In one embodiment the small molecule found to be useful in promoting PE to EP differentiation is gefitinib. In one embodiment gefitinib is used at a concentration of 0.1-100 µM. In one embodiment gefitinib is used at a concentration of 1-10 µM. In one embodiment gefitinib is used at a concentration of 5 µM.

In one embodiment the small molecule found to be useful in promoting PE to EP differentiation is JNK inhibitor VIII. In one embodiment JNK inhibitor VIII is used at a concentration of 0.1-100 µM. In one embodiment JNK inhibitor VIII is used at a concentration of 1-10 µM. In one embodiment JNK inhibitor VIII is used at a concentration of 10 µM.

In one embodiment the small molecule found to be useful in promoting PE to EP differentiation is DNA-PK inhibitor V. In one embodiment DNA-PK inhibitor V is used at a concentration of 0.1-100 µM. In one embodiment DNA-PK inhibitor V is used at a concentration of 1-10 µM. In one embodiment DNA-PK inhibitor V is used at a concentration of 5 µM.

In one embodiment the small molecule found to be useful in promoting PE to EP differentiation is DAPT. In one embodiment DAPT is used at a concentration of 0.1-100 µM.

In one embodiment DAPT is used at a concentration of 1-10 µM. In one embodiment DAPT is used at a concentration of 2.5 µM.

In one embodiment the small molecules found to be useful in promoting PE to EP differentiation is a combination of gefitinib and JNK inhibitor VIII. In one embodiment JNK inhibitor VIII and gefitinib are used at a concentration of 0.1-100 µM. In one embodiment JNK inhibitor VIII and gefitinib are used at a concentration of 1-10 µM. In one embodiment JNK inhibitor VIII and gefitinib are used at a concentration of 5 and 10 µM, respectively.

In one embodiment the concentration of gefitinib is approximately twice that of JNK inhibitor VIII.

In one embodiment one or more small molecules are added in addition to JNK inhibitor VIII and gefitinib.

In one embodiment DAPT is used together with gefitinib and JNK inhibitor VIII in any of the above mentioned DAPT concentrations such as 2.5 µM.

In one embodiment DNA-PK inhibitor V is used together with gefitinib and JNK inhibitor VIII in any of the above mentioned DNA-PK inhibitor V concentrations such as 5 µM.

In one embodiment gefitinib, JNK inhibitor VIII and DNA-PK inhibitor V are used in combination at an individual concentration of 0.1-100 µM. In one embodiment gefitinib, JNK inhibitor VIII and DNA-PK inhibitor V are used in combination at an individual concentration of 1-10 µM.

In one embodiment 1-100 µM gefitinib, 1-100 µM JNK inhibitor VIII, 0.5-50 µM DAPT and 1-100 µM DNA-PK inhibitor V is used. In one embodiment 1-10 µM gefitinib, 5-20 µM JNK inhibitor VIII, 1-10 µM DAPT and 1-10 µM DNA-PK inhibitor V is used in combination. In one embodiment 5 µM gefitinib, 10 µM JNK inhibitor VIII and 5 µM DNA-PK inhibitor V are used in combination.

In one embodiment DAPT, DNA-PK inhibitor V, gefitinib and JNK inhibitor VIII are used in combination in any of the above mentioned concentrations for the respective compounds.

In one embodiment gefitinib, JNK inhibitor VIII, DAPT and DNA-PK inhibitor V are used in combination at an individual concentration of 0.1-100 µM. In one embodiment gefitinib, JNK inhibitor VIII, DAPT and DNA-PK inhibitor V are used in combination at an individual concentration of 1-10 µM. In one embodiment 5 µM gefitinib, 10 µM JNK inhibitor VIII, 2.5 µM DAPT and 5 µM DNA-PK inhibitor V are used in combination.

In one embodiment one or more of the following compounds is used to differentiate PE to EP; Rockout, BPIQ-I, PD174265, p38 inhibitor III, PD169316, DMBI, Syk inhibitor, PD98059, DNA-PK inhibitor V, TGF-β RI inhibitor III, L-685,458, Compound E. In one embodiment Rockout is used at a concentration of 5-10 µM. In one embodiment p38 inhibitor III is used at a concentration of 5-10 µM. In one embodiment PD169316 is used at a concentration of 1-5 µM.

In one embodiment DMBI is used at a concentration of 1-50 µM. In one embodiment DMBI is used at a concentration of 10 µM.

In one embodiment Syk inhibitor is used at a concentration of 1-50 µM. In one embodiment Syk inhibitor is used at a concentration of 1 µM.

In one embodiment BPIQ-I is used at a concentration of 0.1-100 µM.

In one embodiment BPIQ-I is used at a concentration of 1-50 µM. In one embodiment BPIQ-I is used at a concentration of 10 µM.

In one embodiment PD174265 is used at a concentration of 0.1-100 µM. In one embodiment PD174265 is used at a concentration of 1-50 µM. In one embodiment PD174265 is used at a concentration of 10 µM. In one embodiment PD174265 is used at a concentration of 1 µM.

In one embodiment DNA-PK inhibitor V is used at a concentration of 0.1-100 µM. In one embodiment DNA-PK inhibitor V is used at a concentration of 1-10 µM. In one embodiment DNA-PK inhibitor V is used at a concentration of 5 µM.

In one embodiment TGF-β RI inhibitor III is used at a concentration of 0.1-100 µM.

In one embodiment TGF-β RI inhibitor III is used at a concentration of 1-10 µM. In one embodiment TGF-β RI inhibitor III is used at a concentration of 5 µM.

In one embodiment L6 is used at a concentration of 0.1-100 µM. In one embodiment L6 is used at a concentration of 1-10 µM. In one embodiment L6 is used at a concentration of 5 µM.

In one embodiment Compound E is used at a concentration of 50 nM - 5 µM. In one embodiment Compound E is used at a concentration of 100 nM - 1 µM. In one embodiment Compound E is used at a concentration of 500 nM.

In one embodiment, the EP cells obtainable by the method according to the invention are insulin producing cells, optionally together with cells differentiated towards glucagon, somatostatin, pancreatic polypeptide, and/or ghrelin producing cells.

In one embodiment, the cell population comprising EP cells is obtained from a somatic cell population. In some aspects the somatic cell population has been induced to de-differentiate in to an embryonic-like stem cell (i.e. pluripotent). Such de-differentiated cells are also termed induced pluripotent stem cells (iPS).

In one embodiment, the cell population comprising EP cells is in turn obtained from embryonic stem cells.

In one embodiment, the cell population comprising EP cells is in turn obtained from hiPS cells.

In one embodiment differentiation takes place in embryoid bodies and/or in monolayer cell cultures or a combination thereof.

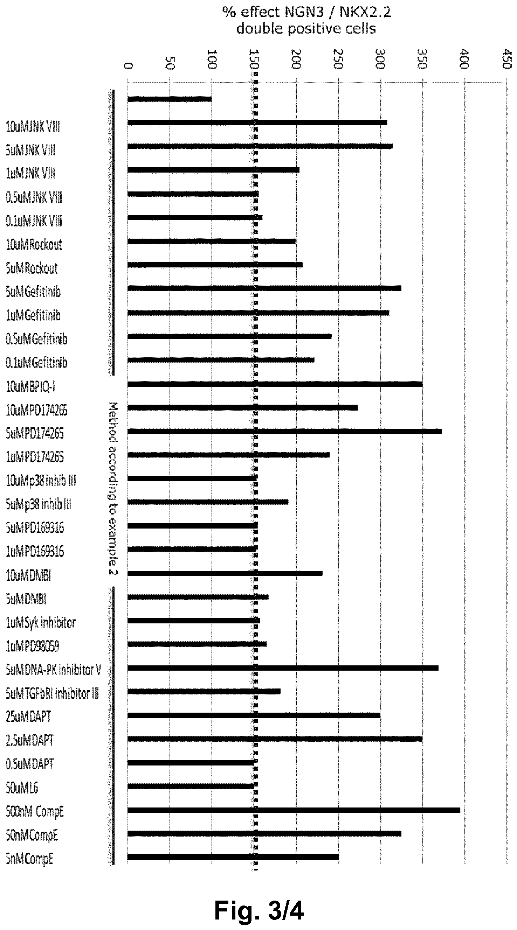

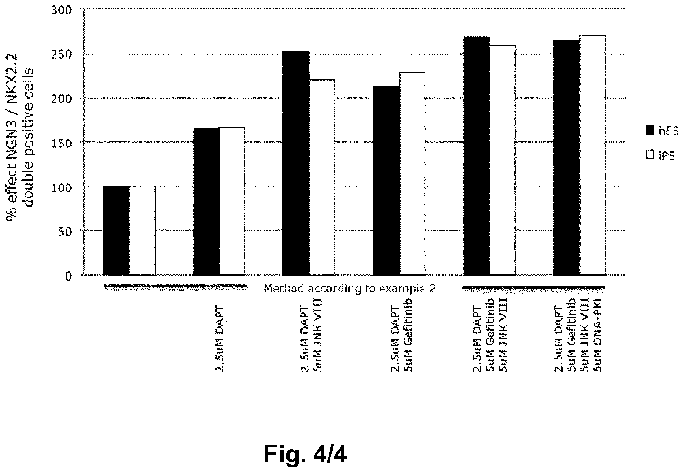

Reference examples of the effect of using said small molecules to increase the amount of NGN3/NKX2.2 double positive cells are shown in the examples of the present document and Figs. 3 and 4.

In one embodiment cells undergoing differentiation into NGN3/NKX2.2 double positive endocrine progenitor cells are treated with said small molecules.

In one embodiment, the method according to reference example 2, with addition of said small molecules, produces a population of endocrine progenitor cells that are 150-400% effect NGN3/NKX2.2 double positive.

In one embodiment, the method according to reference example 2, with addition of said small molecules, produces a population of endocrine progenitor cells that are 150-300% effect NGN3/NKX2.2 double positive.

In one embodiment, the method according to reference example 2, with addition of said small molecules, produces a population of endocrine progenitor cells that are 150-300% effect NGN3/NKX2.2 double positive.

In one embodiment, the method according to reference example 2, with addition of said small molecules, produces a population of endocrine progenitor cells that are at least 150% effect NGN3/NKX2.2 double positive.

In one embodiment, the method according to reference example 2, with addition of said small molecules, produces a population of endocrine progenitor cells that are at least 200% effect NGN3/NKX2.2 double positive.

In one embodiment, the method according to reference example 2, with addition of said small molecules, produces a population of endocrine progenitor cells that are at least 300% effect NGN3/NKX2.2 double positive.

In one embodiment, the method according to reference example 2, with addition of said small molecules, produces a population of endocrine progenitor cells that are up to 400% effect NGN3/NKX2.2 double positive.

In one embodiment, the method according to reference example 2, with addition of said small molecules, the present description produces a population of endocrine progenitor cells that are up to 400% effect NGN3/NKX2.2 double positive.

In one embodiment cells undergoing differentiation into NGN3/NKX2.2 double positive endocrine progenitor cells are exposed to a TGF-β type I receptor inhibitor, a BMP antagonist, an adenylate cyclase activator and nicotinamide in basal medium prior to being treated with said small molecules.

In one embodiment TGF-β type I receptor inhibitor is SB431542 and the BMP antagonist is noggin.

In one embodiment the adenylate cyclase activator is forskolin.

Further embodiments of the invention:
Embodiment 1: A method for obtaining NGN3/NKX2.2 double positive endocrine progenitor cells wherein a cell population comprising pancreatic endoderm cells are exposed to
   a TGF-β type I receptor inhibitor, and
   a BMP antagonist, and
   an adenylate cyclase activator, and
   nicotinamide
   in basal medium.
Embodiment 2: A method according to embodiment 1 wherein the TGF-β type I receptor inhibitor is SB431542 and the BMP antagonist is noggin.
Embodiment 3: A method according to embodiments 1 or 2 wherein the adenylate cyclase activator is forskolin.
Embodiment 4: A method according to embodiments 1-3 wherein the basal medium is RPMI1640.
Embodiment 5: The method according to embodiments 1-4 wherein endocrine progenitor cells are furthermore exposed to DNA-PK inhibitor V, gefitinib, JNK inhibitor VIII or DAPT or any combination of said molecules.
Embodiment 6: The method according to embodiments 1-4 wherein gefitinib, JNK inhibitor VIII and DNA-PK inhibitor V are combined.
Embodiment 7: The method according to embodiments 1-4 wherein gefitinib, JNK inhibitor VIII and DAPT are combined.
Embodiment 8: The method according to embodiments 1-4 wherein gefitinib, JNK inhibitor VIII and DNA-PK inhibitor V are combined.
Embodiment 9: The method according to embodiments 1-4 wherein 1-100 µM gefitinib, 1-100 µM JNK inhibitor VIII, 0.5-50 µM DAPT and 1-100 µM DNA-PK inhibitor V is used.
Embodiment 10: The method according to embodiment 9 wherein 1-10 µM gefitinib, 5-20 µM JNK inhibitor VIII, 1-10 µM DAPT and 1-10 µM DNA-PK inhibitor V is used. Embodiment 11: Cells obtained according to the method of embodiments 1-8. Embodiment 12: Cells obtained according to embodiments 1-8 for use in medicine.
Embodiment 13: Cells obtained according to embodiments 1-8 for use in the treatment of diabetes.
Embodiment 14: Use of cell obtained according to the methods of embodiments 1-8 for the treatment of diabetes.
Embodiment 15: A method for obtaining NGN3/NKX2.2 double positive endocrine progenitor cells wherein a cell population comprising pancreatic endoderm cells are exposed to gefitinib, JNK inhibitor VIII and DAPT.

Surprisingly, in relation to differentiating PE to EP cells, an advantageous effect can be achieved by combining steps from two published protocols known to be suitable for differentiating PE cells to EP cells. It has also surprisingly been shown that certain small molecules and combinations thereof can further drive differentiation of PE to EP and thereby increase the yield of EP cells (leading to a higher percentage of EP in the cell population prepared using the methods of the present invention).

### EXAMPLES

### LIST OF ABBREVIATIONS

- DAPT:: Difluorophenylacetyl)-alanyl-phenylglycine-t-butyl-ester
- DMBI:: (Z)-3-[4-(Dimethylamino)benzylidenyl]indolin-2-one
- CompE:: Compound E
- DE:: Definitive Endoderm
- DEF-CS:: DEF culturing system
- DNA-Pki:: DNA-PK inhibitor V
- EP:: Endocrine Progenitor
- hBS:: human Blastocyst derived Stem
- hES:: human Embryonic Stem
- hESC:: human Embryonic Stem Cell
- hiPS:: human induced Pluripotent Stem
- HSC:: Hematopoietic Stem Cell
- iPS:: Induced Pluripotent Stem
- iPSC:: Induced Pluripotent Stem Cell
- KOSR:: KnockOut™ Serum Replacement
- PE:: Pancreatic Endoderm
- PEST:: Penicillin Streptomycin
- SC:: Stem Cell
- Rockout:: Rho Kinase Inhibitor III

### Reference Example 1: Preparation of PE cell population

hES cells (DEF-hES SA121) or iPS cells (DEF-CHiPS2) were cultured in DEF media (Cellectis) supplemented with 30 ng/ml bFGF (Peprotech #100-18B) and 10 ng/ml noggin (Peprotech #120-10C). DEF medium or DEF-CS medium/system is a defined balanced culture medium for the establishment and propagation of human pluripotent stem cells, DEF-CS medium/system.

The hES cells were differentiated into DE in T75 flasks using the following protocol: Confluent cultures were washed once in RPMI1640 (Gibco #61870) and treated with 3µM CHIR99021 (Axon#1386) in RPMI1640, 0.1% PEST (Gibco #15140). After 24 hours the cells were washed with RPM11640, 0.1% PEST and treated with 100 ng/ml Activin A (Peprotech #120-14E) in RPM11640, 0.1% PEST. 24 hours later, 2% B27 (Invitrogen #17504-044) was added to the ActivinA media for 2 days with daily media change. Cells were maintained at 37°C and 5% CO₂ in a humidified incubator during the differentiation.

DE cells were trypsinized using Tryple Select (Invitrogen #12563-029) and reseeded as single cells in RPMI1640 supplemented with 100 ng/ml ActivinA , 2% B27 and 0.1% PEST in optical 96-well multidishes at 200K/cm² (Corning#3340).

DE cells were allowed to attach and differentiated into PE cells using the following protocol: DE cultures were washed once and treated with 50 nM LDN-193189 (Stemgent#04-0074) in RPMI 1640, 0.1% PEST, 12% KOSR (Gibco # 10828) . After four days the cells were washed with RPMI1640 and differentiated for seven days with 1 µM AM580 (Enzo#BML-GR104), 10 µM JNK inhibitor II (Calbiochem#420119), 50nM LDN-193189 and 64ng/mL bFGF in RPM11640, 0.1% PEST, 12% KOSR. Cells were maintained at 37°C and 5% CO₂ in a humidified incubator during differentiation with daily media change.

### Reference Example 2: Differentiation of PE to EP using a combination protocol

PE cells obtained according to reference example 1 were differentiated for three days with 6 µM SB4311542 (Sigma #S4317), 50 ng/ml noggin (Peprotech #120-10c), 10 µM forskolin (Sigma #F6886) and 10 mM nicotinamide (Calbiochem #481907) in RPMI1640 0.1% PEST and 2% B27. Cells were maintained at 37°C and 5% CO₂ in a humidified incubator during differentiation with daily media change.

### Reference Example 3: Comparison of the method according to reference example 2 with known protocols

The method according to reference example 2 was compared to the methods described in Kunisada *et al.* (2011) (protocol K) and Nostro *et al.* (2012) (protocol N), respectively.

### Protocol K

PE cells obtained according to reference example 1 were differentiated for three days with 10 µM forskolin (Sigma #F6886) and 10 mM nicotinamide (Calbiochem #481907) in RPMI1640, 0.1% PEST and 2% B27. Cells were maintained at 37°C and 5% CO₂ in a humidified incubator during differentiation with daily media change.

### Protocol N

PE cells obtained according to reference example 1 were differentiated for three days with 6 µM SB4311542 (Sigma #S4317), 50 ng/ml noggin (Peprotech #120-10c) in RPM11640, 0.1% PEST and 2% B27. Cells were maintained at 37°C and 5% CO₂ in a humidified incubator during differentiation with daily media change.

Results were obtained as follows:
Relative gene expression levels for the endocrine progenitor marker *NGN3* in response to 3 days of EP differentiation according to protocol K and protocol N and combined protocol K&N (reference example 2). Gene expression is shown as relative to that present in cultures differentiated in basal medium, which was set to 1.

Cells were harvested at EP day 4 and total RNA was extracted using the RNeasy Plus Mini Kit (Qiagen#74134) and RNA concentrations were measured with the NanoDrop ND-1000 spectrophotometer (Thermo Scientific). RNA was reverse transcribed into cDNA using iScript cDNA Synthesis Kit (Bio-Rad) according to the manufacturer's instructions. Each experiment used a fixed amount of RNA (500ng) for cDNA synthesis. The reaction mixture was incubated for 5 min at 25°C, 30 min at 42°C and 5 min at 85°C. Quantitative real-time polymerase chain reactions (qPCR) were run in duplicates using 1/100 of the cDNA per reaction, Taqman gene expression assays (inventoried primer sets against *NGN3* or the housekeeping gene *GAPDH*) and Taqman fast universal PCR master mix in 10 µl reactions. qPCR was performed on an Mx3005P qPCR System (Agilent) using a fast two-step program: 95°C initial denaturation for 3 minutes followed by 40 cycles at 95°C for 15 seconds and 60°C for 20 seconds. Raw data was exported from the MxPro software and analysed using Microsoft Excel and GraphPad Prism. Relative quantification of gene expression was performed using the comparative cycle threshold (DDCt) method (Schmittgen and Livak, 2008) using *GAPDH* as internal reference.

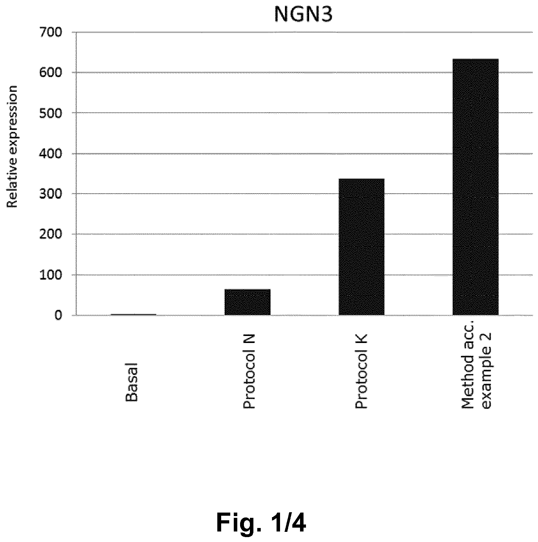

Results in the form of relative NGN3 expression are shown in the below table 1 and in fig. 1. The results show that when combining two individual protocols (protocol K & protocol N) for making endocrine progenitor cells, we can synergistically enhance the level of NGN3 mRNA expression in the EP cell population.

**Table 1: Relative NGN3 expression**

| **Protocol** | **Relative NGN3 mRNA expression** |
|---|---|
| Basal | 1 |
| Protocol N | 63 |
| Protocol K | 338 |
| Method according to reference example 2 | 634 |

After three days of EP differentiation, at EP day 4, media were aspirated followed by fixation of the cells at room temperature for 30 min with 4% paraformaldehyde (VWR, 97.131.000). Cells were washed with PBS and permeabilized with 0.5% Triton X-100 (Sigma, 9002-93-1) for 10 min, washed and blocked in 0.5% TNB-buffer (Perkin Elmer) for 30 min at room temperature. Primary antibodies, sheep anti-NGN3 (R&D systems #AF3444) and rabbit anti-NKX2.2 (Sigma #HPA003468) were diluted 1:1000 and 1:500, respectively, in 0.1% Triton X-100 in PBS and added to each well for overnight incubation at 4°C. Cells were washed three times with PBS. DAPI (4',6-diamidino-2-phenylindole, Applichem, A4099.0010) and secondary antibodies, Alexa Fluor 488 donkey anti-goat and Alexa Fluor 594 donkey anti-rabbit (both Invitrogen) were diluted 1:1000 in 0.1% Triton X-100 in PBS and added to each well for 45 min. Cells were washed five times and left in 200µL PBS for imaging. Imaging was performed using the InCell Analyzer 2000 (GE Healthcare). 4 fields per well with 10x objective were captured. The total cell number based in the DAPI counterstaining and the number of NGN3/NKX2.2 double positive cells was determined using InCell Developer Toolbox 1.8 (GE Healthcare). The fraction of NGN3/NKX2.2 double positive cells were quantified using the InCell developer toolbox software (GE Healthcare) and normalized to the combined protocol K&N (reference example 2) on each plate and the % effect was calculated (% effect NGN3/NKX2.2 double positive = ((S-S_{neg})/(|Sₚₒₛ₋S_{neg}|))*100). Wherein S is % NGN3/NKX2.2 double positive cells for a given compound combination, S_{neg} and Sₚₒₛ are % NGN3/NKX2.2 double positive cells for the negative control and combined protocol K&N, respectively).

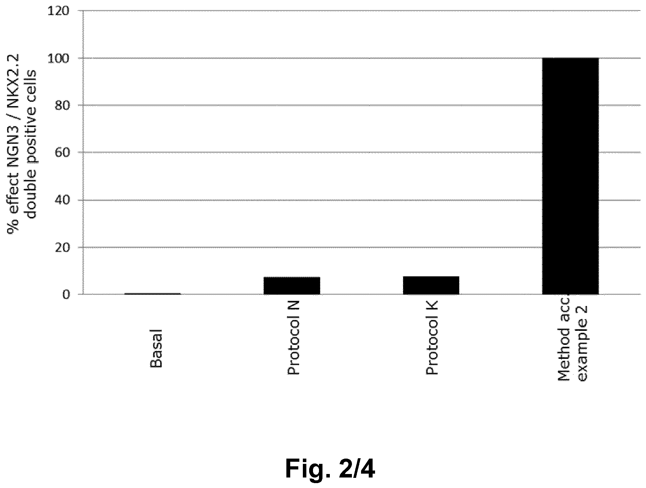

Results in the form of % effect NGN3 / NKX2.2 double positive cells are shown below in table 2 and in fig. 2.

**Table 2: % effect NGN3 / NKX2.2 double positive cells**

| **Protocol** | % effect NGN3 / NKX2.2 double positive cells |
|---|---|
| Basal | 0.1 |
| Protocol N | 7.3 |
| Protocol K | 7.6 |
| Method according to reference example 2 | 100 |

The results show that when combining two individual protocols (protocol K & protocol N) for making endocrine progenitor cells, we can synergistically enhance the level of NGN3 / NKX2.2 double positive cells in the EP cell population.

### Reference Example 4: EP differentiation induced by small molecules

The ability of certain molecules to induce and increase PE to EP differentiation was tested under the following conditions:
An EP cell population was prepared according to reference examples 1 and 2. However, in addition to the reagents used in reference example 2 the small molecules listed in table 3 were added for three days at concentrations specified in table 3 below in concentrations as shown.

After three days of EP differentiation media were aspirated followed by fixation of the cells at room temperature for 30 min with 4% paraformaldehyde (VWR, 97.131.000). Cells were washed with PBS and permeabilized with 0.5% Triton X-100 (Sigma, 9002-93-1) for 10 min, washed and blocked in 0.5% TNB-buffer (Perkin Elmer) for 30 min at room temperature. Primary antibodies, sheep anti-NGN3 (R&D systems #AF3444) and rabbit anti-NKX2.2 (Sigma #HPA003468) were diluted 1:1000 and 1:500, respectively, in 0.1% Triton X-100 in PBS and added to each well for overnight incubation at 4°C. Cells were washed three times with PBS. DAPI (4',6-diamidino-2-phenylindole, Applichem, A4099.0010) and secondary antibodies, Alexa Fluor 488 donkey anti-goat and Alexa Fluor 594 donkey anti-rabbit (both Invitrogen) were diluted 1:1000 in 0.1% Triton X-100 in PBS and added to each well for 45 min. Cells were washed five times and left in 200µL PBS for imaging.

Imaging was performed using the InCell Analyzer 2000 (GE Healthcare). 4 fields per well with 10x objective were captured. The total cell number based in the DAPI counterstaining and the number of NGN3/NKX2.2 double positive cells was determined using InCell Developer Toolbox 1.8 (GE Healthcare). The fraction of NGN3/NKX2.2 double positive cells were quantified using the InCell developer toolbox software (GE Healthcare) and normalized to the combined protocol K&N on each plate and the % effect was calculated (% effect NGN3/NKX2.2 double positive = ((S-S_{neg})/(|Sₚₒₛ-S_{neg}|))*100). Wherein S is % NGN3/NKX2.2 double positive cells for a given compound combination, S_{neg} and Sₚₒₛ are % NGN3/NKX2.2 double positive cells for the negative control and combined protocol K&N, respectively). Values above 150% effect were categorized as hits.

A number of small molecules have been found to promote differentiation of PE cells into EP cells. The molecules are listed in table 3 below.

Furthermore, the efficiency of this combined protocol, measured by NGN3/NKX2.2 double positive cells, can be enhanced even further by addition of small inhibitors that target gamma-secretase, JNK, Rho kinase, P38MAPK, SYK, DNA-PK, PI3K, PDGFR, FGFR or EGFR.

Results are shown in table 4 below and in fig. 3.

**Table 3: Small molecules promoting differentiation**

| **Compound name** | **Target** | **Structure** | **Concentration(s)** | **CAS number** |
|---|---|---|---|---|
| JNK inhibitor VIII | JNK, JNK2, JNK3 | 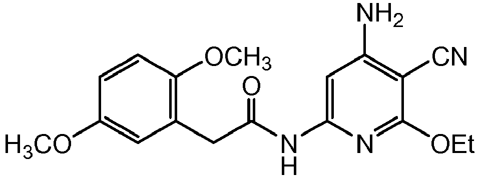 | | 894804-07-0 |
| | | | 10 µM, | |
| | | | 5 µM, | |
| | | | 1 µM, | |
| | | | 0.5 µM, | |
| | | | 0.1 µM | |
| | | | | |
| Rho Kinase Inhibitor III, Rockout | Rho kinase | 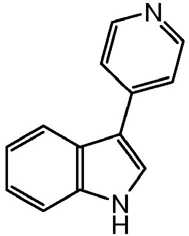 | 10 µM, 5 µM | 7272-84-6 |
| Gefitinib | EGFR | 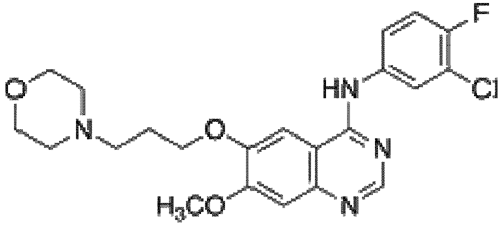 | | 184475-35-2 |
| | | | 5 µM, | |
| | | | 1 µM, | |
| | | | 0.5 µM, | |
| | | | 0.1 µM | |
| | | | | |
| BPIQ-I | EGFR | 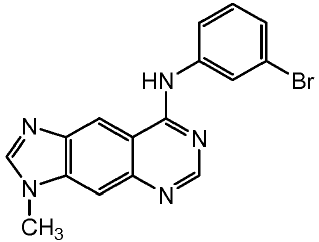 | 10 µM | 174709-30-9 |
| PD174265 | EGFR | 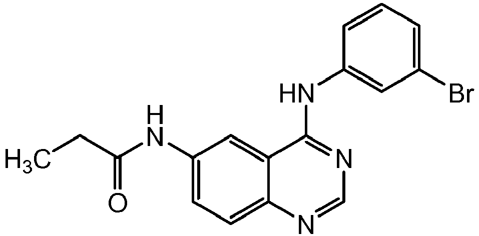 | | 216163-53-0 |
| | | | 10 µM, | |
| | | | 5 µM, | |
| | | | 1 µM | |
| | | | | |
| p38 MAP Kinase Inhibitor III | P38MAPK | 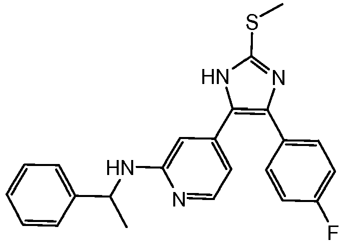 | 10 µM, 5 µM | 581098-48-8 |
| PD169316 | P38MAPK | 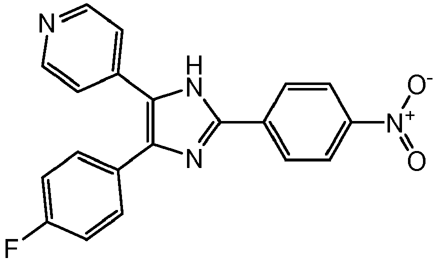 | 5 µM, 1 µM | 152121-53-4 |
| DMBI | PDGFR, FGFR | 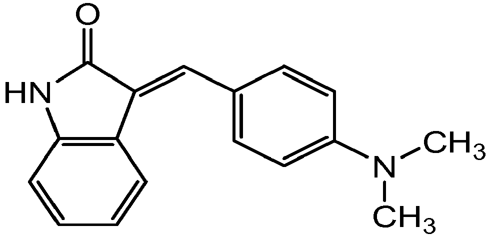 | 10 µM, 5 µM | 07075812 |
| Syk inhibitor | Syk | 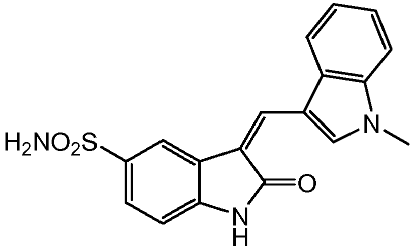 | 1 µM | 622387-85-3 |
| PD98059 | N/A | 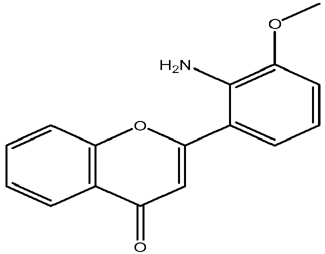 | 1 µM | 167869-21-8 |
| DNA-PK inhibitor V | DNA-PK, PI3K | 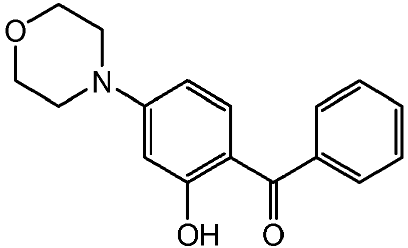 | 5 µM | 404009-46-7 |
| TGF-b RI Inhibitor III | ALK4, ALK5, P38MAPK | 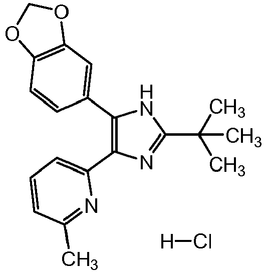 | 5 µM | 356559-13-2 |
| DAPT | Notch | 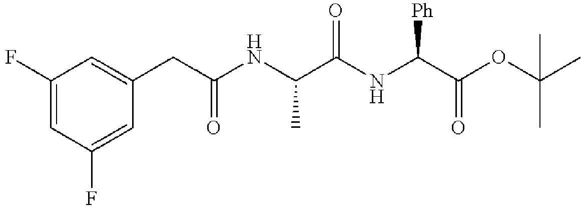 | 25 µM, 2.5 µM, 0.5 µM | 208255-80-5 |
| Compound E | Notch | 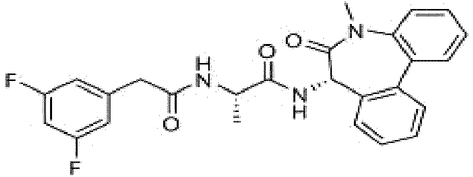 | 500 nM, 50 nM, 5 nM | 209986-17 -4 |
| L-685,458 | Notch | 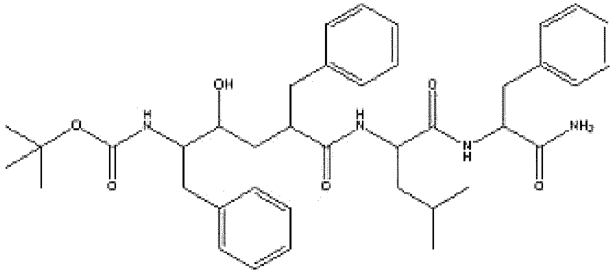 | 50 µM | 292632-98-5 |

**Table 4: % effect NGN3/NKX2.2 double positive cells**

| **Compound** | **% effect NGN3/NKX2.2 double positive cells** |
|---|---|
| Method of Ref. example 2 | 100 |
| Method of Ref. example 2 + 10 µM JNK VIII | 308 |
| Method of Ref. example 2 + 5 µM JNK VIII | 315 |
| Method of Ref. example -2 + 1 µM JNK VIII | 204 |
| Method of Ref. example 2 + 0.5 µM JNK VIII | 156 |
| Method of Ref. example 2 + 0.1 µM JNK VIII | 160 |
| Method of Ref. example 2 + 10 µM Rockout | 199 |
| Method of Ref. example 2 + 5 µM Rockout | 208 |
| Method of Ref. example 2 + 5 µM Gefitinib | 325 |
| Method of Ref. example 2 + 1 µM Gefitinib | 311 |
| Method of Ref. example 2 + 0.5 µM Gefitinib | 242 |
| Method of Ref. example 2 + 0.1 µM Gefitinib | 222 |
| Method of Ref. example 2 + 10 µM BPIQ-I | 350 |
| Method of Ref. example 2 + 10 µM PD174265 | 274 |
| Method of Ref. example 2 + 5 µM PD174265 | 374 |
| Method of Ref. example 2 + 1 µM PD174265 | 240 |
| Method of Ref. example 2 + 10 µM p38 inhibitor III | 153 |
| Method of Ref. example 2 + 5 µM p38 inhibitor III | 191 |
| Method of Ref. example 2 + 5 µM PD169316 | 154 |
| Method of Ref. example 2 + 1 µM PD169316 | 152 |
| Method of Ref. example 2 + 10 µM DMBI | 231 |
| Method of Ref. example 2 + 5 µM DMBI | 167 |
| Method of Ref. example 2 + 1 µM Syk inhibitor | 157 |
| Method of Ref. example 2 + 1 µM PD98059 | 165 |
| Method of Ref. example 2 + 5 µM DNA-PK inhibitor V | 370 |
| Method of Ref. example 2 + 5 µM TGFbRI inhibitor III | 182 |
| Method of Ref. example 2 + 25 µM DAPT | 300 |
| Method of Ref. example 2 + 2.5 µM DAPT | 350 |
| Method of Ref. example 2 + 0.5 µM DAPT | 150 |
| Method of Ref. example 2 + 50 µM L6 | 150 |
| Method of Ref. example 2 + 500 nM CompE | 395 |
| Method of Ref. example 2 + 50 nM CompE | 325 |
| Method of Ref. example 2 + 5nM CompE | 250 |

### Reference Example 5: PE to EP differentiation induced by a combination of small molecules

The ability of certain molecules of table 3 and combinations hereof to induce and increase PE to EP differentiation were tested under the following conditions.

An EP cell population was prepared according to reference example 2. However, in addition to the reagents used in reference example 2, certain small molecules listed in table 3 were added either alone or in combination during the three days of EP differentiation.

After three days of EP differentiation media were aspirated followed by fixation of the cells at room temperature for 30 min with 4% paraformaldehyde (VWR, 97.131.000). Cells were washed with PBS and permeabilized with 0.5% Triton X-100 (Sigma, 9002-93-1) for 10 min, washed and blocked in 0.5% TNB-buffer (Perkin Elmer) for 30 min at room temperature. Primary antibodies, sheep anti-NGN3 (R&D systems #AF3444) and rabbit anti-NKX2.2 (Sigma #HPA003468) were diluted 1:1000 and 1:500, respectively, in 0.1% Triton X-100 in PBS and added to each well for overnight incubation at 4°C. Cells were washed three times with PBS. DAPI (4',6-diamidino-2-phenylindole, Applichem, A4099.0010) and secondary antibodies, Alexa Fluor 488 donkey anti-goat and Alexa Fluor 594 donkey anti-rabbit (both Invitrogen) were diluted 1:1000 in 0.1% Triton X-100 in PBS and added to each well for 45 min. Cells were washed five times and left in 200 µL PBS for imaging.

Imaging was performed using the InCell Analyzer 2000 (GE Healthcare). 4 fields per well with 10x objective were captured. The total cell number based in the DAPI counterstaining and the number of NGN3/NKX2.2 double positive cells was determined using InCell Developer Toolbox 1.8 (GE Healthcare). The fraction of NGN3/NKX2.2 double positive cells were quantified using the InCell developer toolbox software (GE Healthcare) and normalized to the combined protocol K&N on each plate and the % effect was calculated (% effect NGN3/NKX2.2 double positive = ((S-S_{neg})/(|Sₚₒₛ-S_{neg}|))*100). Wherein S is % NGN3/NKX2.2 double positive cells for a given compound combination, S_{neg} and Sₚₒₛ are % NGN3/NKX2.2 double positive cells for the negative control and combined protocol K&N, respectively.

Results in the form of % effect NGN3/NKX2.2 double positive cells are shown below in table 5 and in fig. 4. The results show that the differentiation efficiency of protocol in reference example 2 and 4 is increased when DAPT is added together with JNK inhibitor VIII or Gefitinib or JNK inhibitor VIII plus Gefitinib.

**Table 5: % effect NGN3/NKX2.2 double positive cells.**

| **Compound combination** | **% effect NGN3/NKX2.2 double positive cells** | |
|---|---|---|
| | **SA121 (hES)** | **CHiPS2 (iPS)** |
| Method of reference example 2 | 100 | 100 |
| Method of reference example 2 + 2.5 µM DAPT | 165 | 167 |
| Method of reference example 2 + 2.5 µM DAPT + 5 µM JNK VIII | 252 | 221 |
| Method of reference example 2 + 2.5 µM DAPT + 5 µM Gefitinib | 212 | 229 |
| Method of reference example 2 + 2.5 µM DAPT + 5 µM Gefitinib + 5 µM JNK VIII | 268 | 259 |
| Method of reference example 2 + 2.5 µM DAPT + 5 µM Gefitinib + 5 µM JNK VIII + 5 µM DNA-Pki | 264 | 271 |

While certain features of the invention have been illustrated and described herein, many modifications, substitutions, changes, and equivalents will now occur to those of ordinary skill in the art.

### REFERENCES

Aoi,T., Yae,K., Nakagawa,M., Ichisaka,T., Okita,K., Takahashi,K., Chiba,T., and Yamanaka,S. (2008). Generation of pluripotent stem cells from adult mouse liver and stomach cells. Science 321, 699-702.
Cai,J., Yu,C., Liu,Y., Chen,S., Guo,Y., Yong,J., Lu,W., Ding,M., and Deng,H. (2010). Generation of homogeneous PDX1(+) pancreatic progenitors from human ES cell-derived endoderm cells. J. Mol. Cell Biol. 2, 50-60.
D'Amour,K.A., Bang,A.G., Eliazer,S., Kelly,O.G., Agulnick,A.D., Smart,N.G., Moorman,M.A., Kroon,E., Carpenter,M.K., and Baetge,E.E. (2006). Production of pancreatic hormone-expressing endocrine cells from human embryonic stem cells. Nat. Biotechnol. 24, 1392-1401.
Jiang,J., Au,M., Lu,K., Eshpeter,A., Korbutt,G., Fisk,G., and Majumdar,A.S. (2007). Generation of insulin-producing islet-like clusters from human embryonic stem cells. Stem Cells 25, 1940-1953.
Kroon,E., Martinson,L.A., Kadoya,K., Bang,A.G., Kelly,O.G., Eliazer,S., Young,H., Richardson,M., Smart,N.G., Cunningham,J., Agulnick,A.D., D'Amour,K.A., Carpenter,M.K., and Baetge,E.E. (2008). Pancreatic endoderm derived from human embryonic stem cells generates glucose-responsive insulin-secreting cells in vivo. Nat. Biotechnol. 26, 443-452.
Kunisada,Y., Tsubooka-Yamazoe,N., Shoji,M., and Hosoya,M. (2012). Small molecules induce efficient differentiation into insulin-producing cells from human induced pluripotent stem cells. Stem Cell Res. 8, 274-284.
Nostro,M.C., Sarangi,F., Ogawa,S., Holtzinger,A., Corneo,B., Li,X., Micallef,S.J., Park,I.H., Basford,C., Wheeler,M.B., Daley,G.Q., Elefanty,A.G., Stanley,E.G., and Keller,G. (2011). Stage-specific signaling through TGFbeta family members and WNT regulates patterning and pancreatic specification of human pluripotent stem cells. Development 138, 861-871.
Schulz,T.C., Young,H.Y., Agulnick,A.D., Babin,M.J., Baetge,E.E., Bang,A.G., Bhoumik,A., Cepa,I., Cesario,R.M., Haakmeester,C., Kadoya,K., Kelly,J.R., Kerr,J., Martinson,L.A., McLean,A.B., Moorman,M.A., Payne,J.K., Richardson,M., Ross,K.G., Sherrer,E.S., Song,X., Wilson,A.Z., Brandon,E.P., Green,C.E., Kroon,E.J., Kelly,O.G., D'Amour,K.A., and Robins,A.J. (2012). A scalable system for production of functional pancreatic progenitors from human embryonic stem cells. PLoS. One. 7, e37004.
Takahashi,K., Tanabe,K., Ohnuki,M., Narita,M., Ichisaka,T., Tomoda,K., and Yamanaka,S. (2007). Induction of pluripotent stem cells from adult human fibroblasts by defined factors. Cell 131, 861-872.
Takahashi,K. and Yamanaka,S. (2006). Induction of pluripotent stem cells from mouse embryonic and adult fibroblast cultures by defined factors. Cell 126, 663-676.
Wernig,M., Meissner,A., Foreman,R., Brambrink,T., Ku,M., Hochedlinger,K., Bernstein,B.E., and Jaenisch,R. (2007). In vitro reprogramming of fibroblasts into a pluripotent ES-cell-like state. Nature 448, 318-324.
Zhang,D., Jiang,W., Liu,M., Sui,X., Yin,X., Chen,S., Shi,Y., and Deng,H. (2009). Highly efficient differentiation of human ES cells and iPS cells into mature pancreatic insulin-producing cells. Cell Res. 19, 429-438.

## Claims

1. A method for obtaining NGN3/NKX2.2 double positive endocrine progenitor cells wherein a cell population comprising pancreatic endoderm cells are simultaneously exposed in a basal medium, to:
a) a TGF-β type I receptor inhibitor,
b) a BMP antagonist, wherein the BMP antagonist is noggin,
c) an adenylate cyclase activator, and
d) nicotinamide.

2. The method according to claim 1, wherein the TGF-β type I receptor inhibitor is SB431542.

3. The method according to any one of claims 1 or 2, wherein the adenylate cyclase activator is forskolin.

4. The method according to any one of claims 1-3, wherein said endocrine progenitor cells are at least 8% effect NGN3/NKX2.2 double positive.

5. The method according to any one of claims 1-3, wherein said endocrine progenitor cells are at least 10% effect NGN3/NKX2.2 double positive.

6. The method according to any one of claims 1-3, wherein said endocrine progenitor cells are 10-100% effect NGN3/NKX2.2 double positive.

7. The method according to any one of claims 1-6, wherein the basal medium is RPMI1640.

8. The method according to any one of claims 1-7, wherein endocrine progenitor cells are furthermore exposed to DNA-PK inhibitor V, gefitinib, JNK inhibitor VIII or DAPT or any combination of said molecules.

9. The method according to any one of claims 1-7, wherein gefitinib, JNK inhibitor VIII and DNA-PK inhibitor V are combined.

10. The method according to any one of claims 1-7, wherein gefitinib, JNK inhibitor VIII and DAPT are combined.

11. The method according to any one of claims 1-7, wherein gefitinib, JNK inhibitor VIII, DAPT and DNA-PK inhibitor V are combined.

12. The method according to any one of claims 1-7, wherein 1-100 µM gefitinib, 1-100 µM JNK inhibitor VIII, 0.5-50 µM DAPT and 1-100 µM DNA-PK inhibitor V is used.

13. The method according to any one of claims 8-12, wherein 1-10 µM gefitinib, 5-20 µM JNK inhibitor VIII, 1-10 µM DAPT and 1-10 µM DNA-PK inhibitor V is used.

## Patentansprüche

1. Verfahren zum Erlangen von endokrinen doppelpositiven NGN3/NKX2.2-Progenitorzellen, wobei eine Zellenpopulation, die Entoderm-Bauchspeicheldrüsenzellen umfasst, in einem Grundsubstrat gleichzeitig ausgesetzt werden gegenüber:
a) einem TGF-β-Typ I-Rezeptor-Inhibitor,
b) einem BMP-Antagonist, wobei der BMP-Antagonist Noggin ist,
c) einem Adenylatcyclase-Aktivator und
d) Nicotinsäureamid.

2. Verfahren nach Anspruch 1, wobei der TGF-β-Typ I-Rezeptor-Inhibitor SB431542 ist.

3. Verfahren nach einem der Ansprüche 1 oder 2, wobei der Adenylatcyclase-Aktivator Forskolin ist.

4. Verfahren nach einem der Ansprüche 1 bis 3, wobei die endokrinen Progenitorzellen mit einer Wirkung von mindestens 8 % NGN3/NKX2.2 doppelpositiv sind.

5. Verfahren nach einem der Ansprüche 1 bis 3, wobei die endokrinen Progenitorzellen mit einer Wirkung von mindestens 10 % NGN3/NKX2.2 doppelpositiv sind.

6. Verfahren nach einem der Ansprüche 1 bis 3, wobei die endokrinen Progenitorzellen mit einer Wirkung von 10 bis 100 % NGN3/NKX2.2 doppelpositiv sind.

7. Verfahren nach einem der Ansprüche 1 bis 6, wobei das Grundsubstrat RPMI1640 ist.

8. Verfahren nach einem der Ansprüche 1 bis 7, wobei endokrine Progenitorzellen des Weiteren DNA-PK-Inhibitor V, Gefitinib, JNK-Inhibitor VIII oder DAPT oder irgendeiner Kombination dieser Moleküle ausgesetzt werden.

9. Verfahren nach einem der Ansprüche 1 bis 7, wobei Gefitinib, JNK-Inhibitor VIII und DNA-PK-Inhibitor V kombiniert werden.

10. Verfahren nach einem der Ansprüche 1 bis 7, wobei Gefitinib, JNK-Inhibitor VIII und DAPT kombiniert werden.

11. Verfahren nach einem der Ansprüche 1 bis 7, wobei Gefitinib, JNK-Inhibitor VIII, DAPT und DNA-PK-Inhibitor V kombiniert werden.

12. Verfahren nach einem der Ansprüche 1 bis 7, wobei 1-100 µM Gefitinib, 1 bis 100 µM JNK-Inhibitor VIII, 0,5 bis 50 µM DAPT und 1 bis 100 µM DNA-PK-Inhibitor V verwendet wird.

13. Verfahren nach einem der Ansprüche 8 bis 12, wobei 1 bis 10 µM Gefitinib, 5 bis 20 µM JNK-Inhibitor VIII, 1 bis 10 µM DAPT und 1 bis 10 µM DNA-PK-Inhibitor V verwendet wird.

## Revendications

1. Procédé d'obtention de cellules progénitrices endocrines NGN3/NKX2.2 doublement positives, dans lequel une population cellulaire comprenant des cellules endodermiques pancréatiques est simultanément exposée dans un milieu basal, à :
a) un inhibiteur du récepteur TGF-β de type I,
b) un antagoniste BMP, dans lequel l'antagoniste BMP est noggin,
c) un activateur d'adénylate cyclase, et
d) de nicotinamide.

2. Procédé selon la revendication 1, dans lequel l'inhibiteur du récepteur TGF-β de type I est SB431542.

3. Procédé selon l'une quelconque des revendications 1 ou 2, dans lequel l'activateur d'adénylate cyclase est la forskoline.

4. Procédé selon l'une quelconque des revendications 1 à 3, dans lequel lesdites cellules progénitrices endocrines ont au moins 8 % d'effet NGN3/NKX2.2 double positif.

5. Procédé selon l'une quelconque des revendications 1 à 3, dans lequel lesdites cellules progénitrices endocrines ont au moins 10 % d'effet NGN3/NKX2.2 double positif.

6. Procédé selon l'une quelconque des revendications 1 à 3, dans lequel lesdites cellules progénitrices endocrines ont de 10 à 100 % d'effet NGN3/NKX2.2 double positif.

7. Procédé selon l'une quelconque des revendications 1 à 6, dans lequel le milieu basal est RPMI1640.

8. Procédé selon l'une quelconque des revendications 1 à 7, dans lequel les cellules progénitrices endocrines sont en outre exposées à l'inhibiteur de I'ADN-PK V, au géfitinib, à l'inhibiteur de la JNK VIII ou du DAPT ou à toute combinaison desdites molécules.

9. Procédé selon l'une quelconque des revendications 1 à 7, dans lequel le géfitinib, l'inhibiteur de la JNK VIII et l'inhibiteur de I'ADN-PK V sont combinés.

10. Procédé selon l'une quelconque des revendications 1 à 7, dans lequel le géfitinib, l'inhibiteur de la JNK VIII et le DAPT sont combinés.

11. Procédé selon l'une quelconque des revendications 1 à 7, dans lequel le géfitinib, l'inhibiteur de la JNK VIII, le DAPT et l'inhibiteur de I'ADN-PK V sont combinés.

12. Procédé selon l'une quelconque des revendications 1 à 7, dans lequel 1-100 µM de géfitinib, 1-100 µM de l'inhibiteur de la JNK VIII, 0,5-50 µM de DAPT et 1-100 µM de l'inhibiteur de I'ADN-PK V sont utilisé.

13. Procédé selon l'une quelconque des revendications 8 à 12, dans lequel 1-10 µM de géfitinib, 5-20 µM de l'inhibiteur de la JNK VIII, 1-10 µM de DAPT et 1-10 µM de l'inhibiteur de I'ADN-PK V sont utilisés.
